(19) Europäisches Patentamt
European Patent Office
Office européen des brevets

(11) EP 0 828 506 B1

(12) **EUROPÄISCHE PATENTSCHRIFT**

(45) Veröffentlichungstag und Bekanntmachung des Hinweises auf die Patenterteilung:
**27.02.2002 Patentblatt 2002/09**

(21) Anmeldenummer: **96916137.1**

(22) Anmeldetag: **20.05.1996**

(51) Int Cl.[7]: **A61K 38/15**, A61P 33/10

(86) Internationale Anmeldenummer:
**PCT/EP96/02170**

(87) Internationale Veröffentlichungsnummer:
**WO 96/38165 (05.12.1996 Gazette 1996/53)**

(54) **ENDOPARASITIZIDE MITTEL**

ENDOPARASITICIDAL AGENTS

AGENTS ENDOPARASITICIDES

(84) Benannte Vertragsstaaten:
**AT BE CH DE DK ES FI FR GB GR IE IT LI NL PT SE**

(30) Priorität: **02.06.1995 DE 19520275**

(43) Veröffentlichungstag der Anmeldung:
**18.03.1998 Patentblatt 1998/12**

(73) Patentinhaber: **BAYER AG**
**51368 Leverkusen (DE)**

(72) Erfinder:
- **MENCKE, Norbert**
**51381 Leverkusen (DE)**
- **HARDER, Achim**
**51109 Köln (DE)**
- **JESCHKE, Peter**
**51373 Leverkusen (DE)**
- **HELPAP, Barbara**
**51103 Köln (DE)**

(56) Entgegenhaltungen:

| | |
|---|---|
| **EP-A- 0 059 074** | **EP-A- 0 503 538** |
| **EP-A- 0 662 326** | **WO-A-92/08468** |
| **WO-A-93/19053** | **WO-A-94/19334** |
| **WO-A-95/05181** | **GB-A- 2 252 730** |

- **DATABASE WPI Week 8609 Derwent Publications Ltd., London, GB; AN 86-061936 XP002018637 & ZA,A,8 402 571 (MERCK & CO INC.) , 7.Oktober 1985**
- **ANNU. REP. SANKYO RES. LAB., Bd. 46, 1994, Seiten 67-75, XP002018643 M. KOBAYASHI ET AL.: "Synthesis and anthelmintic activity of the cyclodepsipeptide PF1022A" in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Mischungen von Avermectinen, 22,23-Dihydroavermectinen $B_1$ (Ivermectinen) und Milbemycinen aus der Klasse der makrocyclischen Lactone in Kombination mit cyclischen Depsipeptiden, gegebenenfalls in Gegenwart von Praziquantel oder Epsiprantel, zur Steigerung der endoparasitiziden Wirkung in endoparasitiziden Mitteln.

Gastrointestinale Nematodeninfektionen von Hunden werden in den meisten Fällen von Spezies der drei Nemadodenfamilien Ascarididae, Ancylostomatidae und Trichuridae verursacht. In Katzen sind hauptsächlich die zwei Nemadodenfamilien Ascarididae und Ancylostomatidae weltweit verbreitet. Nach Durchlaufen von verschiedenen Entwicklungsstadien in den unterschiedlichsten Geweben der Wirtstiere kommt es zu einer patenten Infektion des Gastrointestinaltraktes. Während der Präpatenz und Patenz der Infektion verursacht die Parasitose von Rund-, Haken- und Peitschenwürmern erhebliche Probleme speziell bei jungen, heranwachsenden Hunden, Katzen und auch beim Menschen. Eine Therapie oder prophylaktische Behandlung ist daher dringend erforderlich, sowohl zur Heilung bereits erkrankter Tiere als auch zur Gesunderhaltung noch nicht infizierter Tiere.

Der Schutz vor Infektion bei Hund und Katze ist somit als Prophylaxe gegen Humaninfektionen, speziell bei Kindern, sehr bedeutungsvoll.

Eine Reihe nematizider Substanzen werden bereits als Anthelminthika in der Tierzucht verwendet Um einen effektiven Schutz zu erreichen, setzt man neben der reinen Verbindung auch zunehmend Kombinationen mehrerer Substanzen ein.

Allerdings ist die Wirksamkeit dieser vorbekannten Kombinationen, insbesondere bei niedrigen Dosierungen gegen Parasiten nicht immer völlig zufriedenstellend.

Neben den bereits erwähnten gastrointestinalen Helminthenerkrankungen in Hunden und Katzen gibt es weitere schwerwiegende Parasitosen, z.B. Filiariosen, die sehr wirtsspezifisch sind.

Der Parasit Dirofilaria immitis - eine Filarie, endemisch in Teilen von Nord- bis Südamerika, Afrika, Asien aber auch Australien, verursacht die bedeutende kanine- und feline kardiovaskuläre Dirofilariose. Die während der Dirofilaria immitis Infektion von Hunden und Katzen auftretenden schweren pathophysiologischen Veränderungen innerhalb des kardiovaskulären Systems, können einen dramatischen Krankheitsverlauf im Wirtstier bewirken.

Von den bekannten, anthelmintisch aktiven Verbindungen besitzen nur wenige eine Effizienz als Prophylaktikum gegen Dirofilaria immitis.

Anthelminthika, wie beispielsweise Diethylcarbamazin (DEC) sind zwar wirksam, müssen jedoch während einer Übertragung (Mücke) des Erregers täglich verabreicht werden. Mit Einführung der Anthelminthika Ivermectin / Milbemycin aus der Klasse der makrocyclischen Lactone ließ sich die prophylaktische Behandlung der Hunde und Katzen auf eine monatliche Gabe verringern.

Zwar gibt es endoparasitizide Mittel mit hoher Effizenz gegen gastrointestinale Nematoden und andere Mittel mit Wirkung gegen Dirofilaria immitis in Hund und Katze, jedoch zeigt bisher keine der bekannten Verbindungen solch eine Breitspektrum-Wirkung, um als Therapeutikum sowohl gegen alle gastrointestinalen Nematoden als auch als Prophylaktikum gegen Dirofilaria immitis eingesetzt zu werden.

Aus diesem Grunde setzt man verschiedene Wirkstoffkombinationen, die eine verbesserte Wirkung aufweisen und zudem verringerte Nebenwirkungen und Toxizität im Wirt haben, ein.

In den meisten Fällen erfolgt die monatliche Dirofilaria immitis Prophylaxe mit einem Avermectin, wie beispielsweise Ivermectin, aus der Klasse der makrocyclischen Lactone in Kombination mit einem Anthelminthikum, wie Pyrantel oder einem Benzimidazol, z. B. Albendazol (vgl. W. C. Campbell, Ann. Rev. Microbiol. 45 (1991), S. 445-474; J. N. Clark et al. Am. J. Vet. Res. 53(4), (1992), S. 517-520).

Beispielhaft sei eine Kombination bestehend aus 6,0 µg/kg Ivermectin und 5,0 mg/kg Pyrantel-pamoat genannt. Während diese Kombination eine Behandlung und Kontrolle der Ascariden (T. canis und T. leonina) sowie der Hakenwürmer (A. canis und U. steneocephala) gewährleistet, ist sie jedoch nicht gegen Peitschenwürmer (T. vulpis) einsetzbar. Auch das Milbemycin zeigt bei 500 µg/kg eine deutliche Wirkungsschwäche gegen den Hakenwurm Uncinaria stenocephala einem gastrointestinalen Nematoden, der eine schwere Parasitämie in jungen Hunden verursacht (vgl. D. D. Bowman et al. Am. J. Vet. Res. 51 (1990) S. 487; R. Grieve J.Am. Vet. Assoc. 194 (1989), S. 1815).

Darüber hinaus wurde Ivermectin in der Humanmedizin erfolgreich als Mittel gegen Filarieninfektionen und gegen verschiedene gastrointestinale Nematodeninfektionen getestet. Aber auch diese Studien haben gezeigt, daß Ivermectin trotz hoher Dosierung an mehreren aufeinanderfolgenden Tagen keine Wirksamkeit bei Haken- und Peitschenwurm infizierten Patienten hat (vgl. Ottesen & Campbell, J. Antimicrob. Chemother. 34, 1994, S. 195-203).

Diese Gründen verdeutlichen, daß Avermectine, 22,23-Dihydroavermectine $B_1$ (Ivermectine) und Milbemycine aus der Klasse der makrocyclischen Lactone, bisher sowohl singulär als auch in Kombination mit einem Anthelmintikum, nicht gleichzeitig bei niedrigen Dosierungen gegen Rund-, Haken- sowie Peitschenwürmer im Gastrointestinaltrakt von Hunden und Katzen wirken können.

Die vorliegende Erfindung betrifft endoparasitizide Mittel die mindestens ein Avermectin, 22,23- Dihydroaver-

mectin $B_1$ (Ivermectine) oder Milbemycin aus der Klasse der makrocyclischen Lactone in Kombination mit cyclischen Depsipeptiden bestehend aus Aminosäuren und Hydroxycarbonsäuren als Ringbausteine und 6 bis 30 Ringatomen, gegebenenfalls in Gegenwart von Praziquantel oder Epsiprantel, enthalten.

Die erfindungsgemäßen Mittel einer Kombination mindestens eines Avermectins, 22,23 Dihydroavermectins $B_1$ (Ivermectins) oder Milbemycins aus der Klasse der makrocyclischen Lactone mit cyclischen Depsipeptiden bestehend aus Aminosäuren und Hydroxycarbonsäuren als Ringbausteine und 6 bis 30 Ringatomen, weisen einen unerwarteten synergistischen Effekt auf.

Dieser synergistische Effekt im erfindungsgemäßen endoparasitiziden Mittel, hervorgerufen durch die Kombination mindestens eines Avermectins, 22,23-Dihydroavermectins $B_1$ (Ivermectins) oder Milbemycins aus der Klasse der makrocyclischen Lactone mit cyclischen Depsipeptiden bestehend aus Aminosäuren und Hydroxycarbonsäuren als Ringbausteine und 6 bis 30 Ringatomen, bleibt auch in Gegenwart von Praziquantel oder Epsiprantel erhalten.

Avermectine wurden aus dem Mikroorganismus Streptomyces avermitilis als mikrobielle Metabolite isoliert (US-Pat. 4 310 519) und können im wesentlichen als Gemisch, bestehend aus den acht Komponenten $A_{1a}$, $A_{1b}$, $A_{2a}$, $A_{2b}$, $B_{1a}$, $B_{1b}$, $B_{2a}$ und $B_{2b}$, auftreten (I. Putter et al. Experentia 37 (1981) S. 963, Birkhäuser Verlag (Schweiz). Daneben besitzen auch die synthetischen Derivate, insbesondere das 22,23-Dihydroavermectin $B_1$ (Ivermectin), Interesse (US-Pat. 4 199 569). Milbemycin B-41 D konnte ebenso fermentativ aus Streptomyces hygroscopicus isoliert werden (vgl. "Milbemycin: Discovery and Development" I. Junya et al. Annu. Rep. Sankyo Res. Lab. 45 (1993), S. 1-98; JP-Pat. 8 378 549; GB 1 390 336).

Der Einsatz von Avermectinen, 22,23 Dihydroavermectinen $B_1$ (Ivermectinen) und Milbemycinen aus der Klasse der makrocyclischen Lactone als Endoparasitizide ist lange bekannt und Gegenstand zahlreicher Patentanmeldungen sowie Übersichtsartikel (z. B. Biologische Wirkungen in: "Ivermectin and Abamectin" W. C. Campbell, Ed., Springer Verlag, New York, N. Y., 1989; "Avermectins and Milbemycins Part II" H. G. Davies et al. Chem. Soc. Rev. 20 (1991) S. 271-339; Chemische Modifikationen in: G. Lukacs et al. (Eds.), Springer-Verlag, New York, (1990), Chapter 3; Cydectin™ [Moxidectin und Derivate]: G. T. Carter et al. J. Chem. Soc. Chem. Commun. (1987), S. 402-404); EP 423 445-A1). Der Einsatz von Doramectin (Pfizer) als Endoparasitizid ist ebenso bekannt (vgl. "Doramectin - a potent novel endectozide" A. C. Goudie et al. Vet. Parasitol. 49 (1993), S. 5-15).

Ferner sind Kombinationen von Avermectinen, 22,23-Dihydroavermectinen $B_1$ (Ivermectinen) oder Milbemycinen mit bestimmten Anthelminthikaklassen, wie beispielsweise Benzimidazolen, Salizylamiden, Levamisol, Pyrantel oder Praziquantel Gegenstand zahlreicher Patentanmeldungen (z. B.: GB 2 252 730; GB 2 224 933; GB 2 21 3 722; EP-OS 59 074).

Ein cyclisches Depsipeptid PF 1022 A und seine Wirkung gegen Endoparasiten ist bekannt aus EP-OS 382 173 und EP-OS 503 538 (Totalsynthese von PF 1022 A: JP-Pat. 05 229 997; Makoto Ohyama et al., Biosci. Biotech. Biochem. 58 (6), 1994, S. 1193-1194; Makio Kobayshi et al., Annu. Rep. Sankyo Res. Lab. 46, 1994, S. 67- 75; stephen J. Nelson et al., J. Antibiotics 47, (11), 1994, S. 1322-1327).

Weitere cyclische Depsipeptide und ihre endoparasitizide Wirkung sind Gegenstand der folgenden Patentanmeldungen Cyclooktadepsipeptide: WO 93/19053; EP 0 634 408 A1; WO 94/19334; WO 95/07272; EP 626 375; EP 626 376; Cyclohexadepsipeptide: DE-OS 4342 907; WO 93/25543 deutschen Patentanmeldungen P 4 437 198.5; P 4 440 193.0 sowie Cyclotetradepsipeptide: EP-OS 664 297.

Praziquantel 2-(Cyclohexylcarbonyl)-1,2,3,6,7,11b-hexahydro-4H-pyrazino[2,1-a]isochinolin-4-on und seine Wirkung gegen Endoparasiten ist bekannt aus DE-P 2 362 539.

Epsiprantel 2-(Cyclohexylcarbonyl)-2,3,6,7,8,12b-hexahydro-pyrazino[2,1-a]-[2]benzazepin-4(1H)-on und seine Wirkung gegen Endoparasiten ist bekannt aus EP-OS 13 4 984, EP-OS 185 012.

Die Verwendung von Praziquantel und Epsiprantel zur Steigerung der endoparasitiziden Wirkung. von cyclischen Depsipeptiden bestehend aus Aminosäuren und Hydroxycarbonsäuren als Ringbausteine und 6 bis 30 Ringatomen sind Gegenstand einer vorveröffentlichten Patentanmeldung (EP-OS 662 326).

Gegenstand der vorliegenden Erfindung sind daher endoparasitizide Mittel, die mindestens ein Avermectin, 22,23-Dihydroavermectin $B_1$ (Ivermectin) oder Milbemycin aus der Klasse der makrocyclischen Lactone in Kombination mit cyclischen Depsipeptiden bestehend aus Aminosäuren und Hydroxycarbonsäuren als Ringbausteinen und 6 bis 30 Ringatomen, gegebenenfalls in Gegenwart von Praziquantel oder Epsiprantel, enthalten.

Gegenstand der vorliegenden Erfindung ist ferner die Verwendung von Avermectinen, 22,23-Dihydroavermectinen $B_1$ (Ivermectinen) und Milbemycinen aus der Klasse der makrocyclischen Lactone in Kombination mit cyclischen Depsipeptiden bestehend aus Aminosäuren und Hydroxycarbonsäuren als Ringbausteinen und 6 bis 30 Ringatomen, gegebenenfalls in Gegenwart von Praziquantel oder Epsiprantel, zur Herstellung von endparasitiziden Mitteln.

Beispielhaft seien als Kombinationspartner aus der Gruppe der mikrobiellen Metabolite die Avermectine und deren Derivate genannt. Bei diesen Verbindungen handelt es sich um ein Stoffgemisch von makroliden Lactonen der allgemeinen Formel (I)

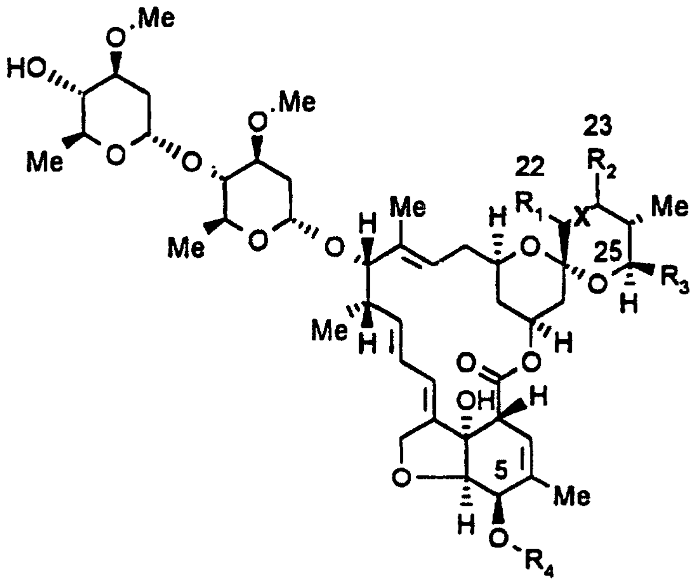

(I)

in welcher
die Reste $R^1$ bis $R^4$ die in der nachfolgenden Tabelle 1 angegebene Bedeutung haben und X für eine Einfach- oder Doppelbindung zwischen der $C_{22}$- und $C_{23}$-Position ($-C_{22}R^1-X-C_{23}R^2-$) stehen kann.

Im Falle einer Doppelbindung befinden sich keine Substituenten ($R^1$, $R^2$) an der $C_{22}$- und $C_{23}$-Position.

Tabelle 1

| Makrocyclisches Lacton | $-C_{22}R^1-X-C_{23}R^2-$ | $R^3$ | $R^4$ |
|---|---|---|---|
| Avermectin $A_{1a}$ | -CH=CH- | -sec-Bu | -Me |
| Avermectin $A_{1b}$ | -CH=CH- | -iso-Pr | -Me |
| Avermectin $A_{2a}$ | $-CH_2-CHOH-$ | -sec-Bu | -Me |
| Avermectin $A_{2b}$ | $-CH_2-CHOH-$ | -iso-Pr | -Me |
| Avermectin $B_{1a}$ | -CH=CH- | -sec-Bu | -H |
| Avermectin $B_{1b}$ | -CH=CH- | -iso-Pr | -H |
| Avermectin $B_{2a}$ | $-CH_2-CHOH-$ | -sec-Bu | -H |
| Avermectin $B_{2b}$ | $-CH_2-CHOH-$ | -iso-Pr | -H |
| 22,23-Dihydroavermectin $B_{1a}$ | $-CH_2-CH_2-$ | -sec-Bu | -H |
| 22,23-Dihydroavermectin $B_{1b}$ | $-CH_2-CH_2-$ | -iso-Pr | -H |
| Doramectin | -CH=CH- | -Chx | -H |
| 22,23-Dihydroavermectin $B_1$ steht für Ivermectin $B_1$;<br>sec-Bu = sekundär Butyl; iso-Pr = Isopropyl; Chx = Cyclohexyl; -Me = Methyl | | | |

Die Avermectine und 22,23-Dihydroavermectine $B_1$ (Ivermectine) der allgemeinen Formel (I) werden in der Regel als Gemische eingesetzt. Von besonderem Interesse ist hierbei das Produkt Abamectin, das im wesentlichen die Avermectine $B_1$ enthält, und deren Hydrierungsprodukte die 22,23-Dihydroavermectine $B_1$ (Ivermectin).

Die mit "b" bezeichneten Verbindungen der makrocyclischen Lactone, die in der $C_{25}$-Position einen iso-Propylrest besitzen, müssen nicht notwendiger Weise von den "a" Verbindungen, welche eine sec-Butylgruppe in der $C_{25}$-Position haben, getrennt werden. Es wird generell das Gemisch beider Substanzen, bestehend aus > 80 % sec-Butylderivat ($B_{1a}$) und < 20 % iso-Propylderivat ($B_{1b}$) isoliert, und kann erfindungsgemäß verwendet werden. Zudem können bei den Stereoisomeren die Substituenten in der $C_{13}$- und $C_{23}$-Position sowohl α- als auch β-ständig am Ringsystem angeordnet sein, d. h. sich oberhalb oder unterhalb der Molekülebene befinden. In jedem Fall werden alle Stereoisomeren erfindungsgemäß berücksichtigt.

Die Milbemycine haben die gleiche makrolide Ringstruktur wie Avermectine oder 22,23-Dihydroavermectine $B_1$ (Ivermectine), tragen aber keinen Substituenten (d.h. fehlendes Oleandrose Disaccharidfragment) in Position 13 ($R^5$ = Wasserstoff).

Beispielhaft seien als Milbemycine aus der Klasse der macrocyclischen Lactone die Verbindungen mit der allgemeinen Formel ( II ) genannt

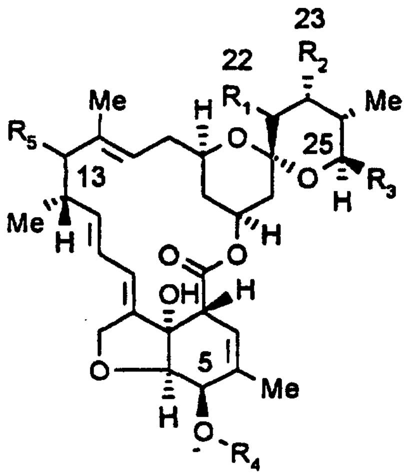

(II)

in welcher
die Reste $R^1$ bis $R^5$ die in der nachfolgenden Tabelle 2 angegebene Bedeutung haben:

**Tabelle 2**

| Makrocyclisches Lacton | $R^1$ | $R^2$ | $R^3$ | $R^4$ | $R^5$ |
|---|---|---|---|---|---|
| Milbemycin B41 D | -H | -H | -iso-Pr | -H | -H |
| Nemadectin | -H | -OH | 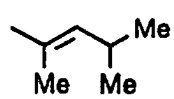 | -H | -H |
| Moxidectin | -H | =N-O-Me | 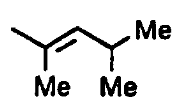 | -H | -H |

iso-Pr = Isopropyl

Von den Kombinationspartnern für die Verbindungen der Formel (I) und (II) sind erfindungsgemäß die nachfolgenden makrocyclischen Lactone von besonderem Interesse:

Avermectin $B_{1a}/B_{1b}$
22,23-Dihydroavermectin $B_{1a}/B_{1b}$ (bzw. Ivermectin $B_{1a}/B_{1b}$)
Doramectin
Moxidectin

Als bevorzugte Kombinationspartner mit Obigen macrocyclischen Lactonen der Formel (I) und (II) sind erfindungsgemäß cyclische Depsipeptide mit 24 Ringatomen zu nennen.

Zu den Depsipeptiden mit 24 Ringatomen zählen Verbindungen der allgemeinen Formel (III)

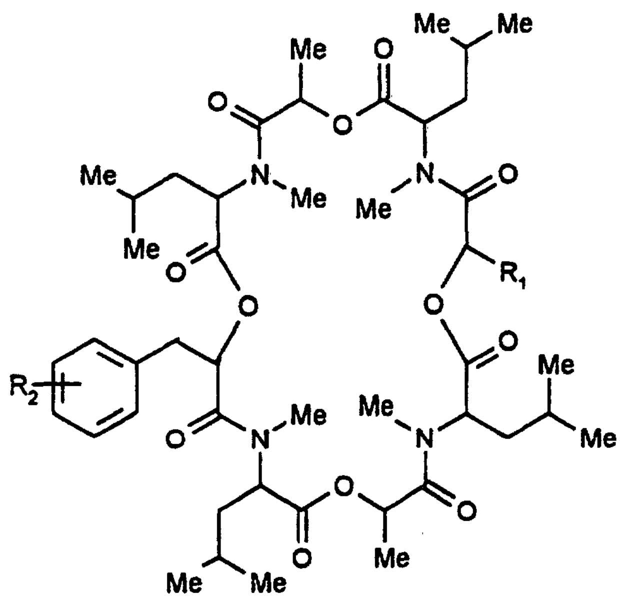

(III)

in welcher

$R^1$ für gegebenenfalls substituiertes Benzyl, wobei als Substituenten genannt seien Wasserstoff, $C_{1-4}$-Alkyl, insbesondere Methyl, Hydroxy, Halogen, insbesondere Fluor, $C_{1-4}$-Alkoxy, insbesondere Methoxy oder tert.-Butyloxy, Nitro, Amino, Dialkylamino, insbesondere Dimethylamino oder Diethylamino, N-Morpholinyl, N-Pyrrolidinyl oder N-Piperidinyl, steht,

$R^2$ für Wasserstoff, $C_{1-4}$-Alkyl, insbesondere Methyl, Hydroxy, Halogen, insbesondere Fluor, $C_{1-4}$-Alkoxy, insbesondere Methoxy oder tert-Butyloxy, Nitro, Amino, Dialkylamino, insbesondere Dimethylamino oder Diethylamino, N-Morpholinyl, N-Pyrrolidinyl oder N-Piperidinyl, steht,

wobei

a) für den Fall, daß $R^1$ für Benzyl steht,

$R^2$ für Wasserstoff, Hydroxy, $C_{1-4}$-Alkoxy, insbesondere Methoxy, Halogen, insbesondere Fluor, Alkenyloxy, insbesondere Allyloxy, steht,

b) für den Fall, daß $R^1$ für Methyl steht,

$R^2$ für Wasserstoff, Hydroxy, $C_{1-6}$-Alkoxy, insbesondere Methoxy, Nitro, Amino, Dialkylamino, insbesondere Dimethylamino, N-Morpholinyl steht.

Im Sinne der vorliegenden Erfindung können alle Verbindungen der allgemeinen Formel (III), die in optisch aktiven, stereoisomeren Formen oder als racemische Gemische vorliegen können, verwendet werden. Vorzugsweise werden jedoch die optisch aktiven, stereoisomeren Formen der Verbindungen der allgemeinen Formel (III) erfindungsgemäß verwendet. Besonders bevorzugt werden die cyclischen Depsipeptide verwendet, die sich aus L-konfigurierten Aminosäuren und D-konfigurierten Hydroxycarbonsäuren als Ringbausteine zusammensetzen.

Beispelhaft sei als cyclisches Depsipeptid die aus EP-OS 382 173 und EP-OS 503 538 bekannte Verbindung PF 1022A der folgenden Formel (IIIa), in der $R^1$ für Benzyl und $R^2$ für Wasserstoff steht, genannt:

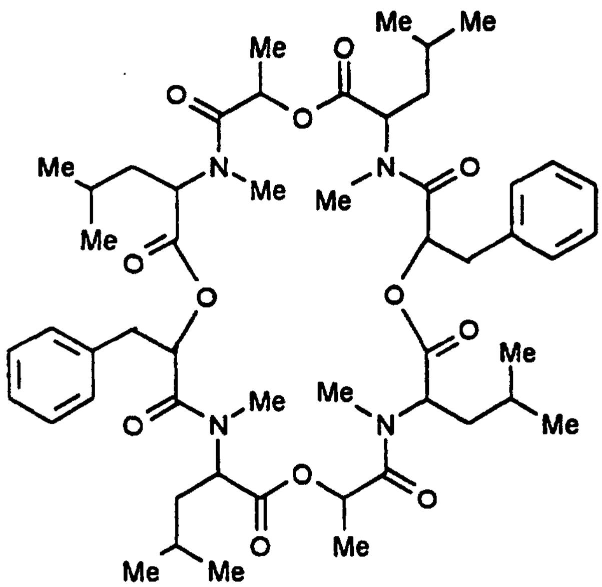

(IIIa)

Außerdem seien als Depsipeptide die aus der PCT-Anmeldung WO 93/19053 und EP 0 634 408 A1 bekannten Verbindungen genannt.

Insbesondere seien aus der PCT-Anmeldung WO 93/19053 und EP 0 634 408 A1 die Verbindungen der folgenden Formel (IIIb), in der $R^1$ für ($R^3$) substituiertes Benzyl steht, genannt:

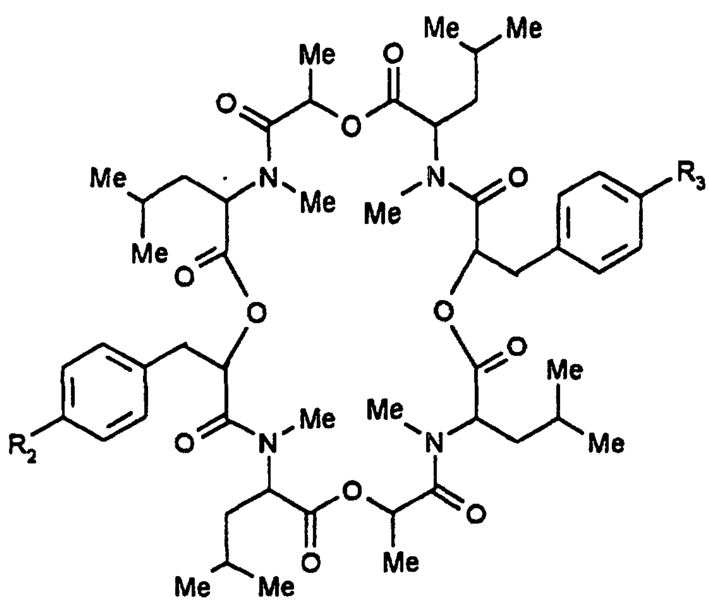

(IIIb)

in welcher

$R^2$ und $R^3$ für N-Morpholinyl, Nitro, Amino, Mono- oder Dimethylamino stehen.

Weiterhin seien als Depsipeptide die aus der PCT-Anmeldung WO 94/19334 bekannten Verbindungen genannt.

Insbesondere seien aus der PCT-Anmeldung WO 94/19334 die Verbindungen der folgenden Formel (IIIc), in der $R^1$ für Benzyl steht, genannt:

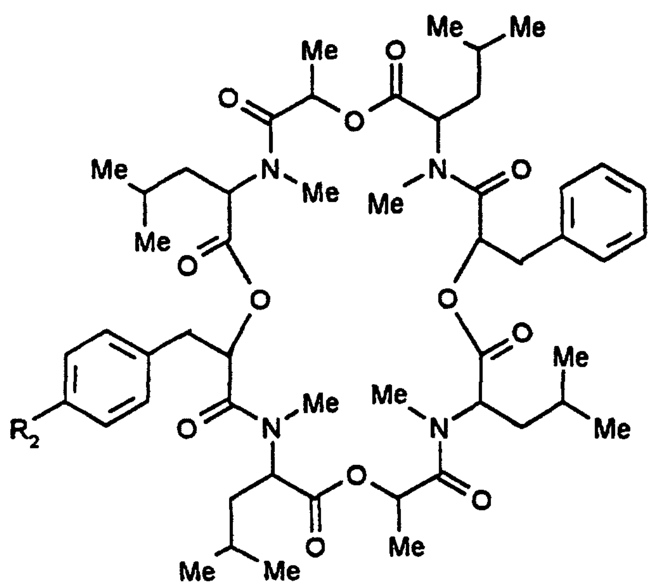

(IIIc)

in welcher

$R^2$ für Hydroxy, Methoxy oder tert.-Butoxy steht.

Schließlich seien als Depsipeptide die aus der PCT-Anmeldung WO 95/07272 bekannten Verbindungen genannt.

Insbesondere seien aus der PCT-Anmeldung WO 95/07272 die Verbindungen der folgenden Formel (IIId) in der $R^1$ für Methyl steht, genannt:

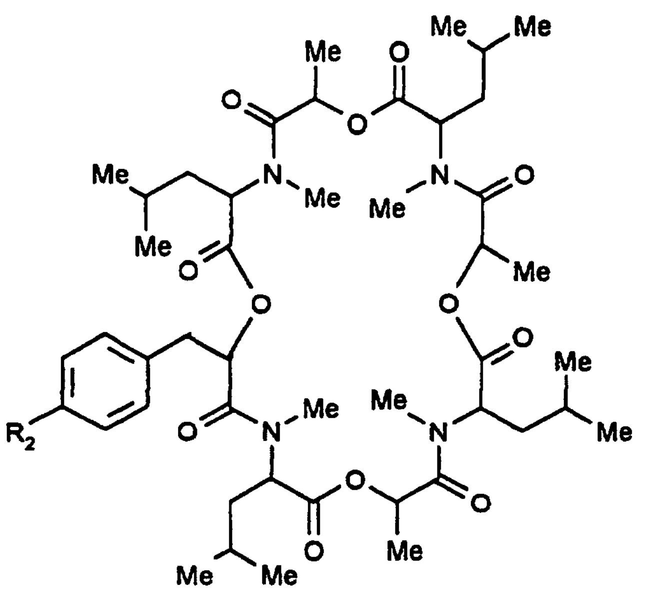

(IIId)

in welcher

$R^2$ für Methoxy, Dimethylamino oder N-Morpholinyl steht.

Nach der am meisten bevorzugten Zusammensetzung der erfindungsgemäßen endoparasitiziden Mittel werden als Kombinationspartner in der vorliegenden Erfindung, die 22, 23-Dihydroavermectine $B_{1a}/B_{1b}$ (Ivermectine $B_{1a}/B_{1b}$) der allgemeinen Formel ( Ia ) aus der Klasse der makrocyclischen Lactone

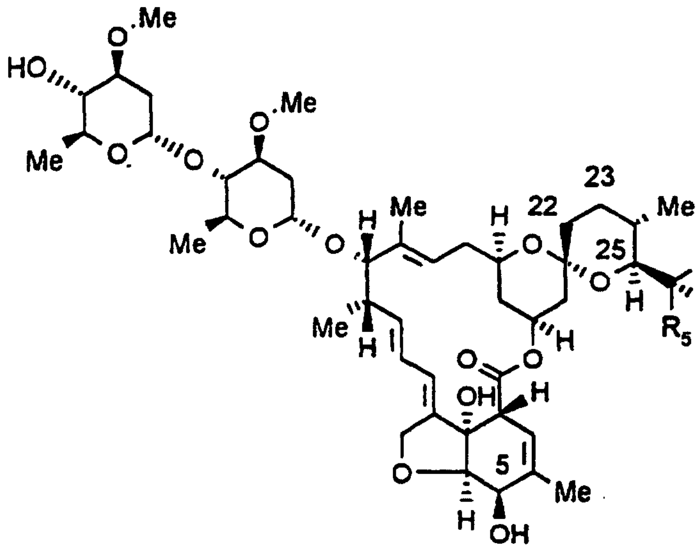

(Ia)

in welcher

$R^5$ für Methyl und Ethyl steht,

mit dem cyclisches Depsipeptid PF 1022A der Formel (IIIa)

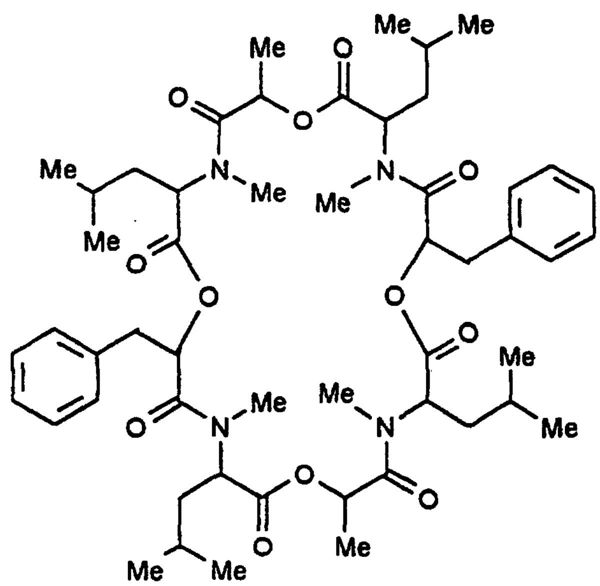

(IIIa)

gegebenenfalls in Gegenwart von Praziquantel oder Epsiprantel, in einem synergistisch wirkenden Verhältnis miteinander kombiniert.

Nach einer weiteren speziellen bevorzugten Zusammensetzung der erfindungsgemäßen endoparasitiziden Mittel werden als Kombinationspartner in der vorliegenden Erfindung, die 22,23-Dihydroavermectine $B_{1a}/B_{1b}$ (Ivermectine $B_{1a}B_{1b}$) der allgemeinen Formel (Ia) aus der Klasse der makrocyclischen Lactone

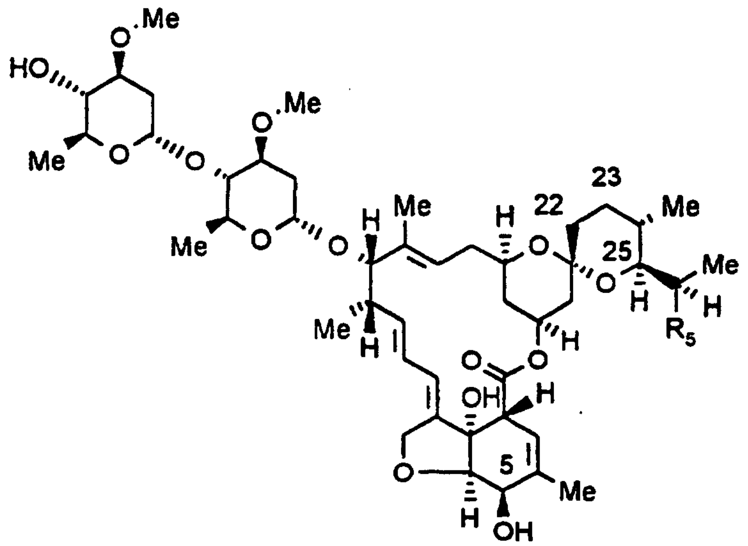

(Ia)

in welcher

$R^5$ für Methyl und Ethyl steht,

mit dem cyclisches Depsipeptid der Formel (IIIb)

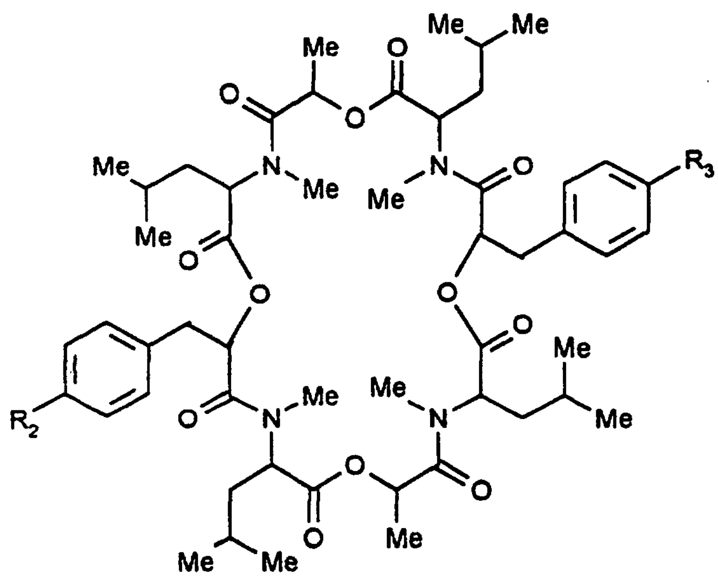

(IIIb)

in welcher

$R^2$ und $R^3$ für N-Morpholinyl steht,

gegebenenfalls in Gegenwart von Praziquantel oder Epsiprantel, in einem synergistisch wirkenden Verhältnis miteinander kombiniert.

Erfindungsgemäß können die Verbindungen der Formel (I) oder (II) und (III) auch mit zwei oder mehreren der aufgezählten Wirkstoffe, gegebenenfalls in Gegenwart von Praziquantel oder Epsiprantel, kombiniert werden.

Die endoparasitizide Wirksamkeit der erfindungsgemäßen Wirkstoffkombinationen liegt deutlich höher als von den Wirkungen der Einzelkomponenten zu erwarten war. Durch Anwendung dieser Kombinationen können daher die Aufwandmenge der Einzelkomponenten reduziert werden. Ihre Anwendung bringt demzufolge ökonomische und

ökologische Vorteile.

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp. Oesophagostomum spp., Chabertia spp., Stephanurus spp., Ancylostoma spp, Uncinaria spp., Bunostomum spp.,

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der. Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur. Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Ol in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase hömogenisiert

Als hydrophobe Phase (Ole) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsauren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten. seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffe,

die gegen pathogene Endoparasiten wirken, vorliegen. Solche Wirkstoffe sind z.B. L-2,3,5,6-Tetrahydro-6-phenyl-imidazothiazol, Benzimidazolcarbamate; Pyrantel.

Anwendungsfertige Zubereitungen enthalten die Wirkstoffe in Konzentrationen von 10 ppm - 20 Gewichtsprozent, bevorzugt von 0,1-10 Gewichtsprozent.

Zubereitungen die vor Anwendung verdünnt werden, enthalten die Wirkstoffe in Konzentrationen von 0,5-90 Gew.-%, bevorzugt von 5 bis 50 Gewichtsprozent.

In den erfindungsgemäßen endoparasitiziden Mitteln wird im Falle einer Anwendung im Hobbytier Hund im allgemeinen ein Gewichtsverhältnis von makrocyclischem Lacton zu Depsipeptid wie 1 zu 500 bis 1000 bevorzugt 1 zu 500 bis 850 ganz be sonder bevorzugt 1 zu 500 eingehalten.

Weiterhin wird in den erfindungsgemäßen endoparasitiziden Mitteln im Falle einer Anwendung im Hobbytier Katze im allgemeinen ein Gewichtsverhältnis von makrocyclischem Lacton zu Depsipeptid wie 1 zu 150 bis 500 bevorzugt 1 zu 150 bis 350 ganz besonder bevorzugt 1 zu 150 bis 200 eingehalten.

Schließlich wird in den erfindungsgemäßen endoparasitiziden Mitteln wird im Falle einer Anwendung im Nutztier im allgemeinen ein Gewichtsverhältnis von makrocyclischem Lacton zu Depsipeptid wie 1 zu 20 bis 400 bevorzugt 1 zu 20 bis 250 ganz besonder bevorzugt 1 zu 20 bis 50 eingehalten.

Im Sinne der vorliegenden Erfindung, können die endoparasitiziden Mittel neben mindestens einem makrocyclischen Lacton und Depsipeptiden auch Praziquantel oder Epsiprantel enthalten. In diesen Fällen wird im allgemeinen ein Gewichtsverhältnis von makrocyclischem Lacton zu Praziquantel oder Epsiprantel in der erfindungsgemäßen Kombination verwendet, welches dem des Depsipeptids entspricht.

## Experimenteller Teil

### Beispiel A

In-vivo Nematodentest

Nematospiroides dubius in der Maus

Mäuse werden experimentell mit Nematoden der Art Nematospiroides dubius infiziert. Zur Infektion wird den Mäusen Nematospiroides dubius oral als 60 filariforme Larven appliziert.

Nach Ablauf der Präpatenzzeit werden die suspendierten Wirkstoffe oral am 12. Tag nach der Infektion appliziert.

Bestimmung der Wirksamkeit:

Die Selektion der Mäuse erfolgt am 20. Tag nach der Infektion. Die Auszählung der adulten Parasiten im *Duodenum* wird mittels Kompressorium durchgeführt. Der Behandlungserfolg in der Dosisgruppe wird ins Verhältnis zur unbehandelten Kontrollgruppe gesetzt.

In den nachfolgenden Tabellen A und B wird die Wirkung der Kombination gegen Nematospiroides dubius in der Maus angegeben.

Tabelle A

Wirkung der Kombination von PF 1022 A und Ivermectin $B_{1a}/B_{1b}$ gegen Nematospiroides dubius in der Maus nach oraler Applikation

| Wirkstoff und Menge [mg/kg] | | Reduktionsrate [%] |
|---|---|---|
| PF 1022 A | 50,0 | 0 |
| Ivermectin $B_{1a}/B_{1b}$ | 0,1 | 0 |
| PF1022 A +<br>Ivermectin $B_{1a}/B_{1b}$ | 50,0<br>0,1 | 100 |
| PF 1022 A | 25,0 | 0 |
| PF1022 A +<br>Ivermectin $B_{1a}/B_{1b}$ | 25,0<br>0,1 | >80 |

Tabelle B

| Wirkung der Kombination von PF 1022 A und Ivermectin $B_{1a}/B_{1b}$ in Gegenwart von Praziquantel gegen Nematospiroides dubius in der Maus nach oraler Applikation | | |
|---|---|---|
| **Wirkstoff und Menge [mg/kg]** | | **Reduktionsrate [%]** |
| PF1022 A +<br>Ivermectin $B_{1a}/B_{1b}$ +<br>Praziquantel | 50,0<br>0,1<br>10,0 | 100 |
| PF1022 A +<br>Ivermectin $B_{1a}/B_{1b}$ +<br>Praziquantel | 50,0<br>0,1<br>5,0 | 100 |
| PF 1022 A +<br>Ivermectin $B_{1a}/B_{1b}$ +<br>Praziquantel | 50,0<br>0,1<br>1,0 | 100 |
| PF1022 A +<br>Ivermectin $B_{1a}/B_{1b}$ +<br>Praziquantel | 25,0<br>0,1<br>10,0 | > 80 |
| PF1022 A +<br>Ivermectin $B_{1a}/B_{1b}$ +<br>Praziquantel | 25,0<br>0,1<br>5,0 | > 80 |
| PF1022 A +<br>Ivermectin $B_{1a}/B_{1b}$ +<br>Praziquantel | 25,0<br>0,1<br>1,0 | > 80 |

**Beispiel B**

In-vivo Nematodentest

Heterakis spumosa in der Maus

Mäuse werden experimentell mit Nematoden der Art Heterakis spumosa infiziert. Zur Infektion wird den Mäusen Heterakis spumosa oral als 90 embryonierte Eier appliziert.

Nach Ablauf der Präpatenzzeit werden die suspendierten Wirkstoffe oral am 46. Tag nach der Infektion appliziert.

Bestimmung der Wirksamkeit:

Die Selektion der Mäuse erfolgt am 54. Tag nach der Infektion. Die Auszählung der adulten Parasiten im Colon und Caecum wird mikroskopisch durchgeführt. Der Behandlungserfolg in der Dosisgruppe wird ins Verhältnis zur unbehandelten Kontrollgruppe gesetzt.

In den nachfolgenden Tabellen C und D wird die Wirkung der Kombination gegen Heterakis spumosa in der Maus angegeben.

Tabelle C

| Wirkung der Kombination von PF 1022 A und Ivermectin $B_{1a}/B_{1b}$ gegen Heterakis spumosa in der Maus nach oraler Applikation | | |
|---|---|---|
| **Wirkstoff und Menge [mg/kg]** | | **Reduktionsrate [%]** |
| PF 1022 A | 50,0 | 0 |
| Ivermectin $B_{1a}/B_{1b}$ | 0,1 | < 50 |
| PF1022 A + | 50,0 | |

Tabelle C (fortgesetzt)

| Wirkung der Kombination von PF 1022 A und Ivermectin $B_{1a}/B_{1b}$ gegen Heterakis spumosa in der Maus nach oraler Applikation | | |
|---|---|---|
| **Wirkstoff und Menge [mg/kg]** | | **Reduktionsrate [%]** |
| Ivermectin $B_{1a}/B_{1b}$ | 0,1 | 100 |
| PF 1022 A | 25,0 | 0 |
| PF1022 A +<br>Ivermectin $B_{1a}/B_{1b}$ | 25,0<br>0,1 | 100 |
| PF 1022 A | 10,0 | 0 |
| PF1022 A +<br>Ivermectin $B_{1a}/B_{1b}$ | 10,0<br>0,1 | > 80 |
| PF 1022 A | 5,0 | 0 |
| PF1022 A +<br>Ivermectin $B_{1a}/B_{1b}$ | 5,0<br>0,1 | > 80 |

Tabelle D

| Wirkung der Kombination von PF 1022 A und Ivermectin $B_{1a}/B_{1b}$ in Gegenwart von Praziquantel gegen Heterakis spumosa in der Maus nach oraler Applikation | | |
|---|---|---|
| **Wirkstoff und Menge [mg/kg]** | | **Reduktionsrate [%]** |
| PF1022 A +<br>Ivermectin $B_{1a}/B_{1b}$ +<br>Praziquantel | 50,0<br>0,1<br>10,0 | 100 |
| PF1022 A +<br>Ivermectin $B_{1a}/B_{1b}$ +<br>Praziquantel | 50,0<br>0,1<br>5,0 | 100 |
| PF1022 A +<br>Ivermectin $B_{1a}/B_{1b}$ +<br>Praziquantel | 50,0<br>0,1<br>1,0 | 100 |
| PF 1022 A +<br>Ivermectin $B_{1a}/B_{1b}$ +<br>Praziquantel | 25,0<br>0,1<br>10,0 | 100 |
| PF1022 A +<br>Ivermectin $B_{1a}/B_{1b}$ +<br>Praziquantel | 25,0<br>0,1<br>5,0 | 100 |
| PF1022 A +<br>Ivermectin $B_{1a}/B_{1b}$ +<br>Praziquantel | 25,0<br>0,1<br>1,0 | 100 |

**Beispiel C**

In vivo Nematodentest

Ancylostoma caninum im Hund

Beagle-Welpen werden experimentell mit Hakenwürmern der Art Ancylostoma caninum infiziert. Zur Infektion wird den Hunden A. caninum oral als 250 $L_3$-Larven appliziert.

Nach Ablauf der präpatenzzeit (oder beim Nachweis eineer Larvenwirksamkeit während der Präpatenzzeit)

werden die Wirkstoffe als reiner Wirkstoff in Gelantinekapseln oral (p.o.) appliziert.
die Wirksamkeit wird nach 2 Methoden bestimmt.

1. Auszählung der mit dem Kot ausgeschiedenen Wurmeier vor und nach der Behandlung.

2. Die prozentuale Wirksamkeit im kritischen Test nach der Formel:

$$\% \text{ Wirksamkeit} = \frac{\text{Abgehende Würmer nach Behandlung}}{\text{Abgehende Würmer nach Behandlung und verbliebene Würmer}} \times 100$$

In der Tabelle wird die wirkung der Kombination gegen Ancylostoma caninum im Hund angegeben.

Tabelle E

| Wirkung der Kombination PF 1002 A und Ivermectin $B_{1a}/B_{1b}$ gegen Ancylostoma caninum im Hund nach oraler Applikation | | |
|---|---|---|
| **Wirkstoff und Menge [mg/kg]** | | **Reduktionsrate [%]** |
| PF 1002 A | 1,0 | 100 |
| | 0,5 | 0 |
| Ivermectin $B_{1a}/B_{1b}$ | 0,01 | 100 |
| | 0,001 | 0 |
| PF 1022 A + | 0,5 | |
| Ivermectin | 0,001 | >80 |

## Patentansprüche

1. Endoparasitizide Mittel die mindestens ein Avermectin, 22,23- Dihydroavermectin $B_1$ (Ivermectine) oder Milbemycin aus der Klasse der makrocyclischen Lactone in Kombination mit cyclischen Depsipeptiden bestehend aus Aminosäuren und Hydroxycarbonsäuren als Ringbausteine und 6 bis 30 Ringatomen, gegebenenfalls in Gegenwart von Praziquantel oder Epsiprantel, enthalten.

## Claims

1. Endoparasiticidal compositions which comprise at least one avermectin, 22,23-dihydroavermectin $B_1$ (ivermectins) or milbemycin from the class of the macrocyclic lactones in combination with cyclic depsipeptides consisting of amino acids and hydroxycarboxylic acids as ring structural units and 6 to 30 ring atoms, optionally in the presence of praziquantel or epsiprantel.

## Revendications

1. Agents endoparasiticides qui contiennent au moins une avermectine, la 22,23-dihydroavermectine $B_1$ (ivermectines) ou une milbemycine de la classe des lactones macrocycliques en combinaison avec des depsipeptides cycliques constitués par des amino-acides et par des acides hydroxycarboxyliques à titre d'éléments constitutifs du noyau et par 6 à 30 atomes nucléaires, le cas échéant en présence de Praziquantel ou d'Epsiprantel.